# EUROPEAN PATENT APPLICATION

(11) **EP 2 360 610 A2**
(43) Date of publication of application: **24.08.2011**
(21) Application number: 11155856.5
(22) Date of filing: 24.02.2011
(51) Int. Cl.: G06F 19/00

(54) **Device for monitoring medication use, method and computer program product**

(30) Priority: 24.02.2010 NL 2004295
(71) Applicant: Van de Geijn Partners B.V., 3992 PK Houten (NL); Verpakapotheek B.V., 2333 CA Leiden (NL)
(72) Inventor: Molijn, Marius Henri Joseph, 3992 PK, Houten (NL)
(74) Representative: Jansen, Cornelis Marinus

(57) **Abstract**

The invention relates to a device for monitoring medication use. The device comprises identification means for identifying a medicine user. In addition, the device comprises identification means for identifying a medication. The device also comprises a processor for verifying whether the identified medication corresponds with a prescribed medication for the identified medicine user. The device further comprises a representation unit for representing a verification signal based on the performed verification.

## Description

The invention relates to a device for monitoring medication use.

In convalescent homes, nursing homes, homes for the elderly and hospitals there is a large-scale use of medication, such as tablets, pills and medically active substances introduced via hypodermic needles.

It goes without saying that the administration of the right medication in the right dose at the right time can be of great importance to the recovery process and/or nursing process of the medicine user. It appears from practice that despite the use of known registration means and the dedication of the nursing staff, there can be a double digit percentage of inaccuracies in the application of the prescribed medication.

The object of the invention is to provide a device for monitoring medication use whereby the risk of incorrect medicine use is reduced. To that end, the device according to the invention comprises identification means for identifying a medicine user, identification means for identifying a medication, a processor for verifying whether the identified medication corresponds with a prescribed medication for the identified medicine user, and a representation unit for representing a verification signal based on the performed verification. The identification means for identifying a medicine user comprise a fingerprint scanner, an iris scanner, a vein pattern scanner and/or a camera with facial recognition software.

Through identification of both the medicine user and the medication, with the aid of verification with the prescribed medication, it can simply be checked whether the match of the user and the medication is correct. By representing a verification signal based on the verification, the verification can be communicated in a simple yet effective manner, which reduces the risk of incorrect administration of medication.

It is greatly preferred that the device comprises a memory for registering the reporting of performed administration operations.

Advantageously, the device can be configured such that a caregiver can be identified too. For instance, a caregiver who is responsible for administering medication can easily be involved in the verification that takes place prior to administration of the medication.

The invention also relates to a method for monitoring medication use.

In addition, the invention relates to a computer program product. A computer program product can comprise a set of computer executable instructions which are stored on an information carrier, such as a CD or a DVD. The set of computer executable instructions which enable a programmable computer to carry out the method as described hereinabove can also be available through downloading from a remote server, for instance via the Internet.

The invention will be elucidated in further detail on the basis of an exemplary embodiment which is represented in the drawing. In the drawing:
Fig. 1 shows a schematic perspective view of a device according to the invention;
Fig. 2 shows a schematic view of a label on a package of medication;
Fig. 3 shows the device of Fig. 1 connected to an electric charger;
Fig. 4 shows a schematic perspective view of a detail of the device of Fig. 1;
Fig. 5 shows a schematic perspective view of a detail of the charger of Fig. 3;
Fig. 6 shows a flow chart of an embodiment of a method according to the invention; and
Fig. 7 shows a system for prescribing, making available and monitoring medication. The Figures are only a schematic representation of a preferred embodiment of the invention. In the Figures, identical or corresponding parts are indicated with the same reference numerals.

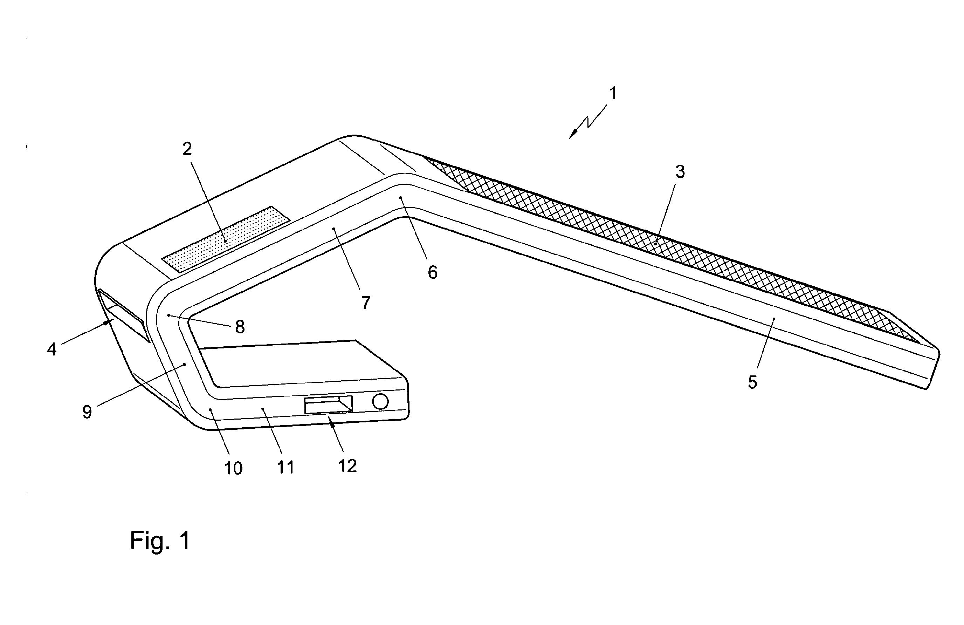

Fig. 1 shows a schematic perspective view of a device 1 according to the invention. The device 1 is integrally formed into a portable unit and has a distinctive form, so that the device can be easily recognized by users. The device 1 serves for monitoring medication use and is provided to that end with a number of components. For instance, the device 1 has identification means for identifying a medicine user, and a display on which information can be shown, especially intended for a caregiver. The identification means are designed as a fingerprint scanner 2 with which the fingerprint of the medicine user can be determined. Naturally, other identification means for identifying a medicine user can be used too, such as an iris scanner, a barcode reader, an Rfid reader or a keyboard for entering a patient code. Also, a vein pattern scanner can be used. The vein pattern scanner, which is also called an artery scanner, vascular pattern recognition (VPR), Near Infrared (NIR) scanner or a vein scanner, can be designed as, for instance, a Finger Vein Scanner or a Vein Palm Scanner. In an advantageous embodiment, the vein pattern scanner is designed as a scanner for scanning a vein pattern which is not located on the hand and/or arm of the patient. For the identification of a user, a vein pattern in the face, for instance on a cheek or forehead, can be scanned. This can be advantageous when identifying a patient who misses one or two hands and/or arms and/or has one or two hands in plaster, or when identifying a patient who can hardly, if at all, move and/or turn his hand and/or arm. Also, a camera with facial recognition software can be used for identifying the medicine user, and, optionally, the caregiver. In a specific embodiment of the device according to the invention, after identification of the medicine user, personal information is represented, for instance a picture of the medicine user, as additional verification possibility.

The device 1 further comprises identification means for identifying a medication, such as a barcode scanner 4 or a camera. The device 1 also comprises a processor (not shown) for verifying whether an identified medication corresponds with a prescribed medication for the identified medicine user. Data of the prescribed medication can be available to the processor in different manners. Data of the prescribed medication can for instance be stored in a memory of the device. Such data can also be transmitted from a central server to the device. Further, such data can be detected by the device, for instance a camera, via information provided on the medication itself, or the package thereof, and be interpreted. In the latter case, the package of the medication is provided with, for instance, a label with a barcode comprising information about the medication itself, and with printed information about the intended medicine user and the physician who has prescribed the medication. The identified medication is then verified, based not on a separate prescription, but based on an identifier which contains information of the medication prescription. In that case, the identifier can be represented on a package manufactured specifically for the medicine user.

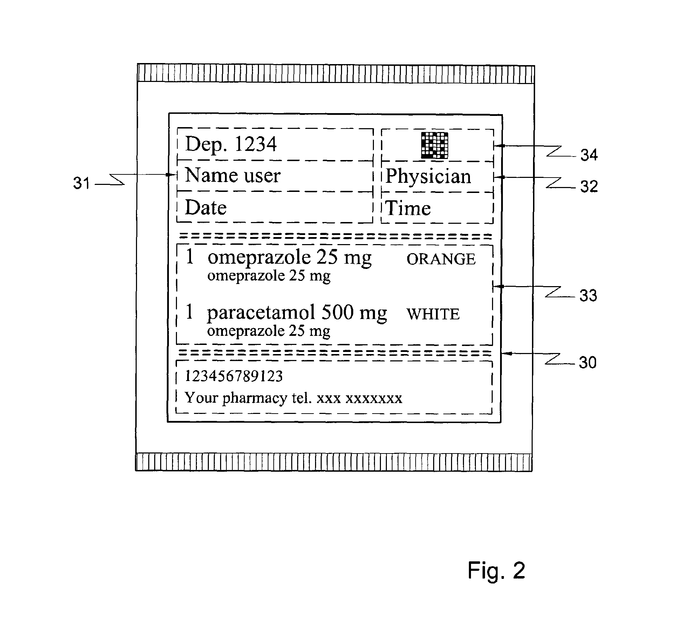

Fig. 2 shows a schematic view of such a label 30 that is provided on a package of a medication. The label 30 shown has a field 31 for stating the name of the medicine user, a field 32 for stating the name of the prescribing physician, and a field 33 for stating the medication, in this case two tablets. In addition, the label comprises a two-dimensional barcode 34. Label 30 also has further fields for representing additional information, such as information about the pharmacy that has provided the medication. It is clear that all sorts of variations are possible when representing information on the label. For instance, also the intended date and time of ingestion of the medication can be given.

The use of the device 1 preferably comprises the identification of a caregiver, also called nurse. It is also preferred that verification be carried out to check whether the identified caregiver involves a pre-registered person who is authorized to administer medication to medicine users. Thus, the role of the responsible person when administering medication can be recorded. Identification of the caregiver is done through, for instance, entering an identification code, by keying in a name and/or taking a fingerprint scan. After identification of the caregiver, the device 1 optionally shows personal data of the caregiver, such as a welcome message with the first name of the caregiver. The display 3 is preferably designed as a touch screen, so that the caregiver can exchange information with the device 1.

When ingesting medication, the intended medicine user is identified, in the preferred embodiment with the fingerprint scanner 2. Also, a medication or a package thereof is identified. In a practical application, the medication or the package thereof is provided with a barcode or another one-dimensional or two-dimensional coding. Then, the processor verifies whether the identified medication corresponds with a medication prescribed by a certified doctor, for instance a physician, for the identified medicine user. Preferably, the processor also verifies whether the medication is to be administered at the actual moment of ingestion. When the medication corresponds with the prescribed medication, the display 3 represents a positive verification signal, for instance the letters "OK". When the identified medication does not correspond with the prescription, the display represents a negative verification signal, for instance the word "ERROR". The intended medicine use can then be corrected, for instance by scanning the correct medication. In a preferred embodiment of the invention, if the display has represented a negative verification signal, this signal can optionally be confirmed by the caregiver, for instance by, once more, taking a fingerprint scan from the caregiver or by keying in a confirmation code. It can thus be confirmed that the rejected ingestion operation of the medication has not been performed. As an option, in a comparable manner, also, an approved ingestion action can be confirmed by the caregiver to the device.

Further, a medication message can be generated comprising data of the identified medicine user and of the identified medication. By transmitting the medication message to a central processing unit, the medication can be tracked and stored, for instance for registration in a database which is available to someone treating the medicine user. As an option, a medication message can be printed, for instance as part of a daily report. The medication message can also be stored in a memory integrated in the device for registering and reporting the performed administration operations.

Preferably, the device 1 is used each time medication is administered, for instance several times a day per medicine user. When verifying the medication, also, the desired time of administration/ingestion can be included.

The preferred embodiment of the device shown in Fig. 1 is formed as a portable unit which comprises a multiple-folded strip-shaped profile. The multiple-folded, strip-shaped profile comprises a first strip segment 5 which is connected via a fold 6 with an obtuse angle, to a second strip-shaped segment 7. This configuration bears some resemblance to a traditional telephone receiver. Here, the first strip-segment 5 is longer than the second strip segment 7, so that the configuration bears some resemblance to a traditional bar scanner. Through the use of an obtuse angle, a device is formed whereby the top surfaces, that is, the first and second segment 5, 7, can be brought at a reduced angle relative to the horizontal plane, while at the same time, the device can be easily operated and/or read by both the medicine user and the caregiver. The first strip segment 5 is provided with the display, 3 and thus makes effective use of the greater length with regard to the second strip segment 7. The second strip segment 7 is further provided with the fingerprint scanner 2 which is positioned such that the thumb is put on the scanner 2 in a natural gesture when the unit is gripped near the second strip segment 7. During use, the second strip segment 7 can be presented to the medicine user, while the first segment 5 stays within the sight of the caregiver. Optionally, the caregiver too can identify himself via the fingerprint scanner 2. Naturally, the caregiver can log in in another manner, for instance by keying in a pin code. From a point of view of safety, the unit 1 can be configured such that the caregiver has to identify himself once more when the unit has not been used for a period of time, for instance five minutes.

The second strip segment 7 is connected via a second fold 8 to a third strip segment 9. Similarly, the third strip segment 9 is connected via a third fold 10 to a fourth, final strip segment 11. The fourth strip segment 11 forms a support when the unit 1 is laid on a flat surface, for instance a table. The bar code scanner 4 or camera is integrated in the third strip segment 9, so that the caregiver can perform the identification of the medication in an ergonomically pleasant manner, that is, by gripping the first, long segment 5. The fourth, final strip segment 11 comprises a chargeable energy source (not shown) for electrically feeding the unit 1. The fourth segment 11 further comprises power points 12 for communication with another device, for instance an electric charger. Thus, in substantially each of the strip segments 5, 7, 9, 11 adjacent the folds 6, 8, 10, an electrical component is provided, so that an efficient housing is combined with an elegant design. Naturally, a specific segment can be provided with more than one component. For instance, both the energy source and the processor may be provided in the fourth segment 11. Also, a segment can optionally be designed having no electrical components.

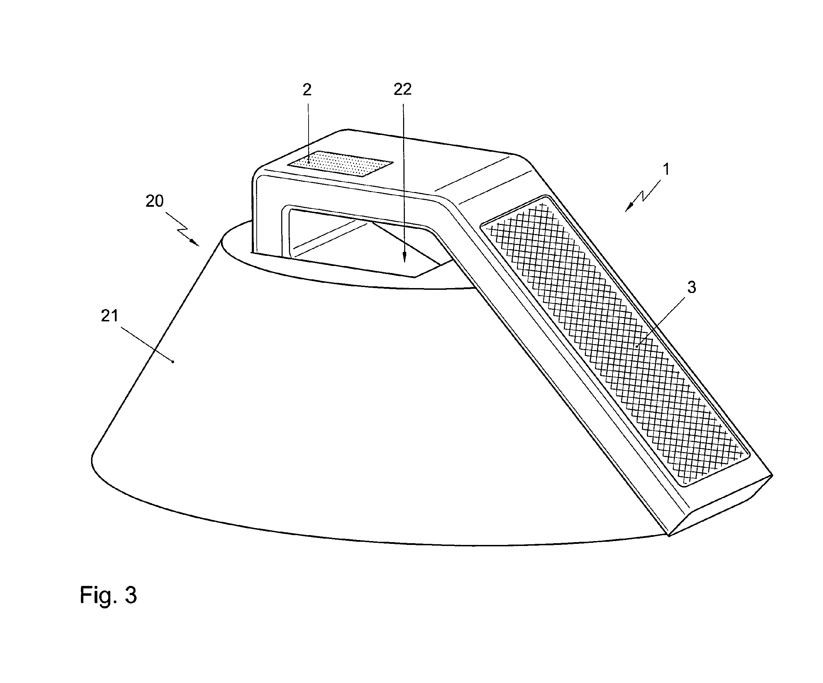

Fig. 3 shows the unit 1 which is connected to an electric charger, designed as docking station 20 with a specific design that corresponds with the design of the portable unit 1. The docking station 20 has approximately the appearance of a truncated cone 21, while its taper end is provided with a cavity 22 for receiving the fourth segment 11 of the unit 1. As a result, a stable position of the unit 1 can be obtained, wherein the first segment 5 abuts against the outer surface of the cone 21. Together, the docking station 20 and the unit 1 form a system.

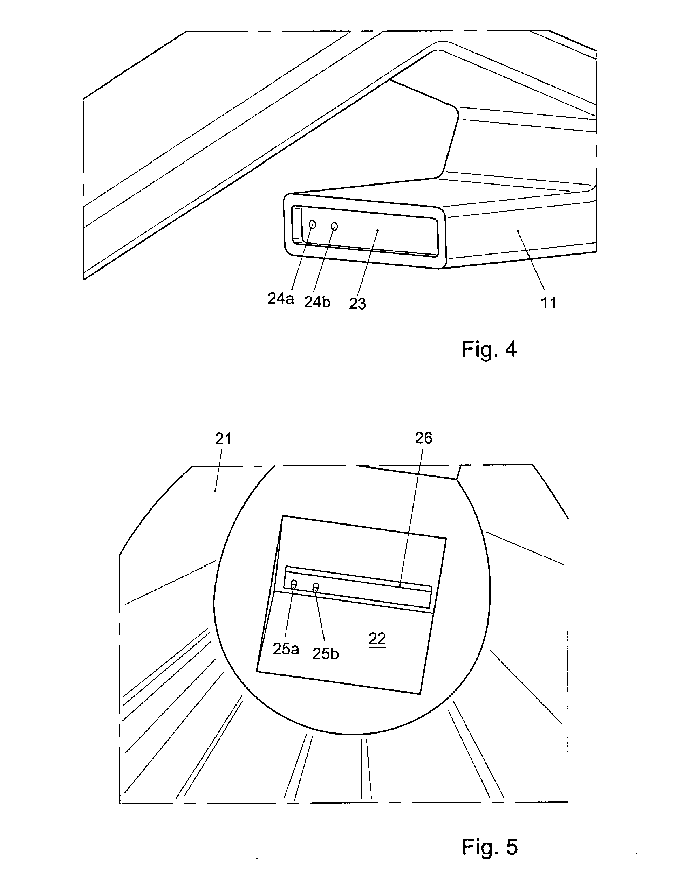

Figs. 4 and 5 show a schematic perspective view of a detail of the unit 1 and the docking station 21, respectively. In Fig. 4, the end face of the extremity of the fourth strip segment 5 is shown. In the extremity, a part 23 is inwardly staggered, matching a projecting part 26 in the cavity 22 of the docking station 20, in order to provide an improved mechanical and electric coupling between the station 20 and the unit 1. The inwardly staggered part 23 and the projecting part 26 are provided with matching electric contact parts for, for instance, charging the chargeable energy source in the unit 1. Also, an electric connection between the unit 1 and the docking station 20 can be used for realizing data transmission, for instance with regard to performed verifications.

It is noted that the portable unit can also be formed differently, with for instance more or fewer than four strip segments, with strip segments which are connected to each other via a right-angled fold or as a singular flat strip, such as the appearance of a PDA. Optionally, the portable unit can further be provided with extendible or roll-up modules.

Owing to the portable design of the unit, this can easily be carried along by a caregiver, together with, for instance, an amount of medications to be administered for a group of medicine users.

In a preferred embodiment, in order to perform the verification, information about prescribed medication is available to the processor of the portable unit 1, preferably via a receiver/transmitter configuration integrated in the unit 1, which can receive information wirelessly from a central database in which prescriptions with prescribed medication are digitally stored. However, it is also possible to store the information about the prescribed medication locally, in advance, in the unit, which reduces data transport. As described hereinabove, the prescribed medication can be represented on the medication or the package thereof in the form of personal data.

The method for monitoring medication use can be carried out by using specific hardware structures, such as FPGA and/or ASIC components. On the other hand, the method can be carried out at least partly by using a computer program product containing instructions which have a processor of a computer system carry out the above-described steps of the method of the invention. In principle, all steps can be carried out on a single core processor. However, it is noted that at least one step can be carried out on a separate processor, for instance the step of verifying whether the identified medication corresponds with a prescribed medication.

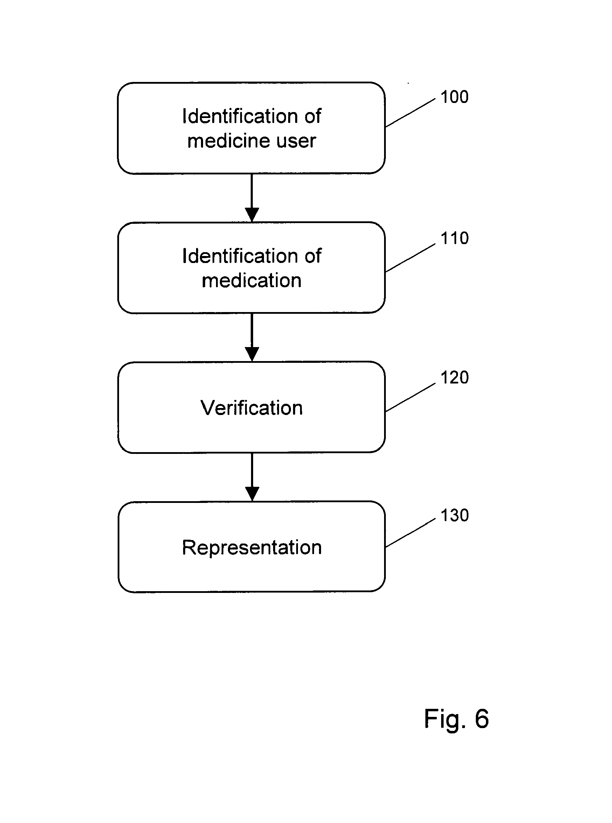

Fig. 6 shows a flow chart of an embodiment of the method according to the invention. The method is utilized for monitoring medication use. The method comprises the steps of identifying 100 a medicine user, identifying 110 a medication, verifying 120 whether the identified medication corresponds with a prescribed medication for the identified medicine user, and representing 130 a verification signal based on the performed verification.

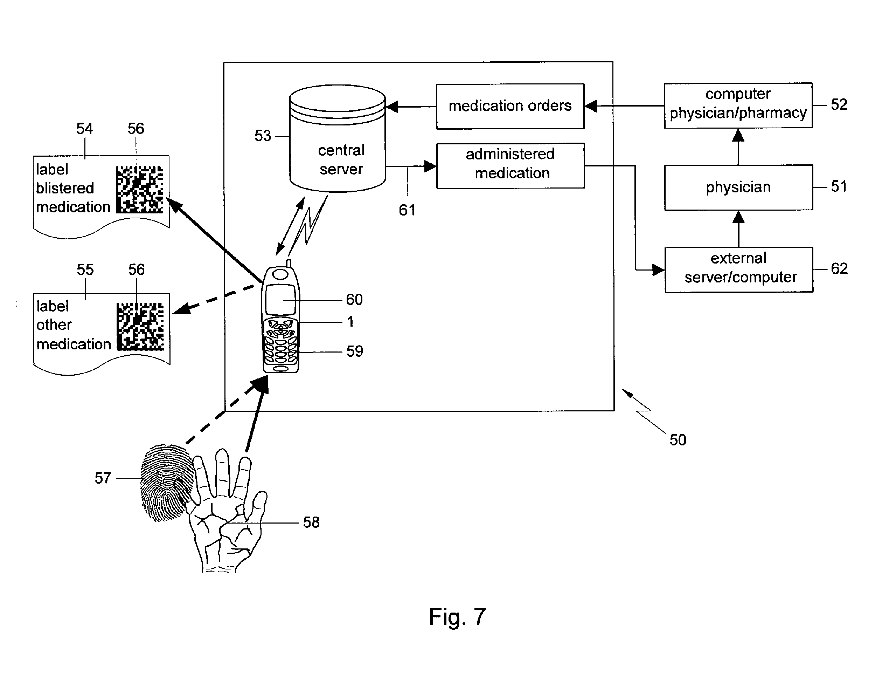

Preferably, the above-described method is utilized in a system. Fig. 7 shows a system 50 for prescribing, making available and monitoring medication. In the system 50, medication can be prescribed, for instance in a prescription of a physician 51. The prescription can for instance be transmitted from a computer 52 of the physician to the central server 53 or central processing unit. The prescription can also be transmitted from the computer of the doctor to a pharmacy or pharmacy information system (AIS), which can then forward the prescription to the central server or processing unit. The prescription can be recorded on the central server or processing unit, while among others, patient identifiers such as name and/or date of birth, and/or coding of the medication, and/or administering instructions such as dosing, and/or periods of administration and/or times of administration and/or specific transmission codes can be recorded.

Information can be transmitted from the central server or processing unit 53 to a medication provider. The medication provider can for instance be a pharmacy, automated packaging company and/or a staff member in a nursing home who puts out and/or prepares medication for a caregiver who will make his round.

As described, data of the prescribed medication can be transmitted from the central server or processing unit 53 to the device 1. This can be done in the form of, for instance, a list of patients and with the medication each of the patients must take.

The caregiver who will administer the medication to patients can proceed to a first patient with a device 1 and with the medication. Preferably, all medication is packaged, per patient, per time of ingestion in one package, such as a blister package 54 or a bag 55. Preferably, the package is provided with an identifier 56 that contains information of the medication prescription, such as the patient identifiers, the coding of the medication, the administering instructions and/or transmission codes. Here, the identifier can be, for instance, a two-dimensional barcode 56, such as, for instance, a QR-code or a MaxiCode.

Once at a patient, the caregiver can administer or give out the medication to the patient after identification of the medicine user, based on, preferably, biometric properties 57, 58 of the patient such as a vein pattern 58 or a fingerprint 57, and after identification of the medication. Optionally, with the aid of, for instance, a keyboard 59 or touch screen 60, the caregiver can then confirm that the medication has indeed been given out and/or ingested by the patient. Information about this confirmation can be stored on the portable unit 2 and/or can be forwarded to the central server 53 or processing unit 53.

Further, it is possible that the central server or processing unit is connected to an interface 61. Via the interface 61, information from the central server or processing unit can be transmitted to, for instance, the computer 52 of the physician or to another external server 62 or computer 62, for instance to an electronic patient file (EPD). It can thus be enabled that for instance a physician, such as the physician 51 who has prescribed the medication, can check whether the medication has indeed been dispensed and/or administered and/or can verify whether the medication has been provided in the prescribed manner.

The invention is not limited to the exemplary embodiments described here. Many variants are possible.

For instance, the representation unit can be implemented not only as display, but also as a different kind of representation unit, for instance a unit for generating an acoustic signal. Also, the representation unit can generate optical as well as acoustic signals, for instance only an acoustic signal when the identified medication does not correspond with the prescribed medication.

It is noted that in a specific embodiment, the device for monitoring medication use does not identify the caregiver, for instance for limiting the functionality of the apparatus and by simplifying the operation, for, for instance, a specific target audience.

Further, specific functionality can be implemented separately, so that instead of an integrally formed device, the arrangement is of modular design. For instance, the fingerprint scanner can be realised separately. As a result, the flexibility of the device with regard to technological updates is improved.

Such variants will be clear to the skilled person and are understood to fall within the scope of the invention, as set forth in the following claims.

## Claims

1. A device for monitoring medication use, comprising:
- identification means for identifying a medicine user;
- identification means for identifying a medication;
- a processor for verifying whether the identified medication corresponds with a prescribed medication for the identified medicine user;
- a representation unit for representing a verification signal based on the performed verification,
wherein the identification means for identifying a medicine user comprise a fingerprint scanner, an iris scanner, a vein pattern scanner and/or a camera with facial recognition software.

2. A device according to claim 1, wherein the identification means for identifying a medicine user comprise a barcode reader, an Rfid-reader or a keyboard.

3. A device according to claim 1 or 2, wherein the identification means for identifying a medication comprise a barcode scanner, an Rfid-reader or a camera.

4. A device according to any one of the preceding claims, further comprising a memory for registering the reporting of performed administering operations.

5. A device according to any one of the preceding claims, wherein the processor has access to a database in which prescriptions for medication are stored.

6. A device according to any one of the preceding claims, wherein the display unit is configured for representing an optical and/or acoustic signal.

7. A device according to any one of the preceding claims, further comprising identification means for identifying a caregiver.

8. A device according to any one of the preceding claims which is integrally formed into a portable unit.

9. A device according to any one of the preceding claims, wherein the portable unit comprises a multiple-folded, strip-shaped profile.

10. A device according to any one of the preceding claims, wherein substantially each of the strip segments contiguous to the folds is provided with an electrical component.

11. A device according to any one of the preceding claims, wherein the multiple-folded, strip-shaped profile comprises a first strip segment which is connected via a fold with an obtuse angle, to a second strip segment.

12. A device according to claim 11, wherein the first strip segment is longer than the second strip segment.

13. A device according to claim 11 or 12, wherein the first strip segment is provided with a monitor and wherein the second strip segment is provided with the identification means for identifying the medicine user.

14. A device according to any one of the preceding claims, further comprising a chargeable energy source for connection to an electric charger.

15. A device according to any one of the preceding claims, further comprising a transmitter/receiver combination for wireless communication with a central processing unit.

16. A method for monitoring medication use, comprising the steps of:
- identifying a medicine user;
- identifying a medication;
- verifying whether the identified medication corresponds with a prescribed medication for the identified medicine user; and
- representing a verification signal based on the performed verification, wherein during identification of a medicine user, use is made of a fingerprint scanner, an iris scanner, a vein pattern scanner and/or a camera with facial recognition software.

17. A method according to claim 16, further comprising the step of identifying a caregiver.

18. A method according to claim 17, further comprising the step of verifying the caregiver.

19. A method according to any one of claims 16-18, further comprising the steps of:
- generating a medication message comprising data of the identified medicine user and of the identified medication; and
- transmitting the medication message to a central processing unit for processing in a database.

20. A method according to any one of claims 16 - 19, further comprising the step of registering a performed or not performed administering operation of a medication.

21. A method according to any one of claims 16 - 20, further comprising the step of forwarding the registration to the central processing unit.

22. A method according to any one of claims 16 - 21, further comprising the step of representing an image of the medicine user after identification of the medicine user.

23. A computer program product comprising instructions for having a processor carry out the method of monitoring medication use, wherein the method comprises the steps of:
- identifying a medicine user;
- identifying a medication;
- verifying whether the identified medication corresponds with a prescribed medication for the identified medicine user; and
- representing a verification signal based on the performed verification, wherein during identification of a medicine user, use is made of a fingerprint scanner, an iris scanner, a vein pattern scanner and/or a camera with facial recognition software.
